# EUROPEAN PATENT APPLICATION

(11) **EP 2 690 440 A1**
(43) Date of publication of application: **29.01.2014**
(21) Application number: 12177887.2
(22) Date of filing: 25.07.2012
(51) Int. Cl.: G01N 33/68

(54) **Protein level of C9ORF72 for diagnosing a neurodegenerative disease**

(71) Applicant: Institut du Cerveau et de la Moelle Épinière-ICM, 75013 Paris (FR); Assistance Publique-Hôpitaux de Paris (APHP), 75001 Paris (FR); Centre National de la Recherche Scientifique (CNRS), 75116 Paris (FR); INSERM (Institut National de la Santé et de la Recherche Médicale), 75013 Paris (FR); Université Pierre et Marie Curie - Paris 6 (UMPC), 75005 Paris (FR)
(72) Inventor: Le Ber, Isabelle, 75014 Paris (FR); Lamari, Foudil, 94000 Créteil (FR)
(74) Representative: Icosa

(57) **Abstract**

The present invention relates to a method for diagnosing a neurodegenerative disease in a subject, or determining a predisposition of a subject to a neurodegenerative disease or determining an increased risk of a subject of developing a neurodegenerative disease, wherein said method comprises determining the protein level of C9ORF72 in a body fluid of the subject, and comparing said protein level to a reference value.

## Description

### FIELD OF INVENTION

The present invention relates to the diagnosis of a neurodegenerative disease in a subject. More precisely, the present invention relates to a method for the diagnosis of a neurodegenerative disease, such as, for example, frontotemporal lobar degeneration (FTLD), amyotrophic lateral sclerosis (ALS) or FTLD-ALS in a subject, wherein said method comprises measuring the protein level of C9ORF72 in a biological fluid of the subject, such as, for example, in plasma or in cerebrospinal fluid.

### BACKGROUND OF INVENTION

Neurodegenerative disease is a general term designating diseases characterized by a progressive loss of structure or of function of neurons, including death of neurons. Symptoms of neurodegenerative diseases are highly dependent on the impacted neurons, and may thus be completely different. Indeed, if motor neurons are compromised, motor functions will be affected, and symptoms such as, for example, tremor, respiratory compromise and the like will be encountered. However, if neurons involved in cognitive functions are affected, the neurodegenerative disease may cause memory, attention, or language problems. This loss of global cognitive ability is usually referred as dementia.

Among the large list of neurodegenerative diseases, frontotemporal dementia (FTD) and amyotrophic lateral sclerosis (ALS) are both devastating neurological diseases, with totally different symptoms. FTD is, after Alzheimer's disease, the second most common cause of presenile dementia. FTD patients experience a progressive degeneration of the frontal and temporal lobes of the brain, inducing changes in personality, behavior and language, but no motor dysfunctions. On the other side, ALS patients usually do not present cognitive impairment, but the degeneration of upper and lower motor neurons in ALS gives rise to progressive spasticity, muscle wasting and weakness.

Despite these apparently unrelated symptoms, the existence of kindreds comprising patients diagnosed with ALS, FTD or both with ALS and FTD supports the implication of a common genetic cause to both diseases.

WO2008/095261 describes the possible implication of the 9p21 locus in the development of neurodegenerative disorders, such as FTD and motor neuron disease. Moreover, WO2008/095261 describes a method for diagnosing a neurodegenerative disease, said method comprising the detection of a marker linked to 9p21 locus.

In 2011, a non-coding hexanucleotide (GGGGCC) repeat expansion in the first codon of the C9ORF72 gene was identified as a common genetic abnormality responsible for ASL and FTD (DeJesus-Hernandez et al, Neuron, 2011; Renton et al, Neuron, 2011). This repeat expansion may be the most common genetic abnormality in both ALS and FTD. Recent studies also suggested an implication of this repeat expansion in other neurodegenerative diseases, such as, for example, schizophrenic and psychotic disorders (Snowden et al, Brain, 2012).

The number of hexanucleotide repeats is lower than 24 in healthy controls, and may reach up to 1600 in FTD or ALS patients (De Jesus-Hernandez et al, Neuron, 2011). The detection of the hexanucleotide repeat expansion in the C9ORF72 gene may thus allow diagnosing a neurodegenerative disease in a subject.

Two methods may be used in order to detect said repeat expansion. The first one is repeat-primed PCR. Although rapid to carry out, this method presents the drawback to be not accurate enough for clinical diagnosis. Indeed, the maximum of GGGGCC repeats detectable by repeat-primed PCR is about 60, and using this method, one cannot precisely quantify the number of hexanucleotide repeats (De Jesus-Hernandez et al, Neuron, 2011; Renton et al, Neuron, 2011). The second method allowing the detection of hexanucleotide expansion is Southern Blot. This method, unlike repeat-primed PCR, makes it possible to detect long size expansions. However, Southern Blot is a cost- and time-consuming method.

There is thus a need for a rapid, cost-effective and accurate method for detecting hexanucleotide repeat expansion. Said method may thus allow a pre-selection of patients potentially having a repeat expansion, before submitting said preselected patients to a Southern Blot analysis in order to confirm the presence of said repeat expansion, and consequently, to diagnose said patients for the presence of ALS and/or FTD.

The Inventors herein show that the presence of the hexanucleotide repeat expansion leads to a significant reduction of the protein level of C9ORF72 detected in the plasma and in the cerebrospinal fluid. Therefore, the detection of the C9ORF72 protein in body fluids of a subject may represent a relatively simple and cost effective laboratory test to identify patients likely to have hexanucleotide repeat expansion in the C9ORF72 gene. Those with positive or borderline result could then have Southern Blot analysis to confirm the presence of the hexanucleotide repeat expansion.

### SUMMARY

One object of the invention is a method for diagnosing a neurodegenerative disease in a subject, or determining a predisposition of a subject to a neurodegenerative disease or determining an increased risk of a subject of developing a neurodegenerative disease, wherein said method comprises determining the protein level of C9ORF72 in a body fluid of the subject, and comparing said protein level to a reference value. In one embodiment, said body fluid is selected from plasma or cerebrospinal fluid. In another embodiment, the protein level is measured by immunoassay, preferably by ELISA. In another embodiment, a reduced protein level as compared to the reference value is indicative of the presence of; of a predisposition to; or of an increased risk of the subject developing a neurodegenerative disease. In another embodiment, the neurodegenerative disease is frontotemporal dementia, progressive non-fluent aphasia, semantic dementia, frontotemporal dementia associated with amyotrophic lateral sclerosis, frontotemporal lobar degeneration associated with amyotrophic lateral sclerosis, amyotrophic lateral sclerosis without dementia, spastic paraplegia, primary lateral sclerosis, schizophrenia, psychotic disorders, Alzheimer's disease, idiopathic Parkinson, Parkinson-dementia, preogressive supranuclear palsy, corticobasal degeneration syndromes, Lewy bodies dementia and multiple system atrophy. In another embodiment, the degenerative disease is frontotemporal dementia (FTD), frontotemporal lobar degeneration (FTLD), amyotrophic lateral sclerosis (ALS), FTD-ALS or FTLD-ALS. In another embodiment, the subject presents a familial history of neurodegenerative disease. In another embodiment, the subject does not present any familial history of neurodegenerative disease.

Another object of the invention is a method for classifying a subject as having a neurodegenerative disease or a predisposition of developing a neurodegenerative disease or as being at risk of developing a neurodegenerative disease, wherein said method comprises determining the protein level of C9ORF72 in a body fluid of the subject, and comparing said protein level to a reference value.

Another object of the invention is a prescreening method for identifying subjects for whom the assessment of the presence of a hexanucleotide repeat expansion in the C9ORF72 gene may be beneficial, said method comprising determining the protein level of C9ORF72 in a body fluid of the subject, and comparing said protein level to a reference value.

Another object of the invention is a method for determining the risk of neurodegenerative disease onset in a subject, wherein said method comprises determining the protein level of C9ORF72 in a body fluid of the subject, and comparing said protein level to a reference value.

Another object of the invention is a method for monitoring the response to a treatment and/or the efficacy of the treatment in a subject having or being at risk of having a neurodegenerative disease, wherein said method comprises determining the protein level of C9ORF72 in a body fluid of the subject, and comparing said protein level to a reference value.

Another object of the invention is a method for monitoring the progression of a neurodegenerative disease in a subject carrying a C9ORF72 hexanucleotide repeat expansion, wherein said method comprises determining the protein level of C9ORF72 in a body fluid of the subject, and comparing said protein level to a reference value.

In one embodiment, the reference value is derived from the measurement of the protein level of C9ORF72 in a control sample derived from one or more substantially healthy subjects. In another embodiment, the reference value corresponds to the level of C9ORF72 protein previously measured in a body fluid of said subject.

### DETAILED DESCRIPTION

The present invention relates to a method for diagnosing a neurodegenerative disease in a subject, or determining a predisposition of a subject to a neurodegenerative disease or determining an increased risk of a subject of developing neurodegenerative disease, wherein said method comprises determining the protein level of C9ORF72 in a body fluid of the subject, and comparing said protein level to a reference value.

The present invention also relates to a method for monitoring the progression of a neurodegenerative disease in a subject, said method comprising determining the protein level of C9ORF72 in a body fluid of the subject, and comparing said protein level to a reference value.

As used herein, "C9ORF72" stands for Chromosome 9 Open Reading Frame 72. This gene (Accession Number: NG_031977.1) gives rise to a transcript (Accession number NM_018325.2) having the sequence SEQ ID NO: 1 and encoding a protein having the sequence SEQ ID NO: 2.

In one embodiment, said neurodegenerative disease is a C9ORF72-associated neurodegenerative disease.

Examples of C9ORF72-associated neurodegenerative diseases include, but are not limited to, frontotemporal dementia (FTD), behavioral variant FTD, progressive non-fluent aphasia, semantic dementia, frontotemporal dementia associated with amyotrophic lateral sclerosis (FTD-ALS), frontotemporal lobar degeneration (FTLD), frontotemporal lobar degeneration associated with amyotrophic lateral sclerosis (FTLD-ALS), amyotrophic lateral sclerosis (ALS) without dementia (also referred to as Gehrig' disease), spastic paraplegia, primary lateral sclerosis, schizophrenia, psychotic disorders, Alzheimer's disease, idiopathic Parkinson, Parkinson-dementia, progressive supranuclear palsy, corticobasal degeneration syndromes, Lewy bodies dementia and multiple system atrophy (De Jesus-Hernandez et al, Neuron, 2011; Renton et al, Neuron, 2011; Snowden et al, Brain, 2012; Lindquist et al, Clinical Genetics, 2012; Majounie et al, New England Journal of Medicine, 2012).

In one embodiment, said neurodegenerative disease is dementia. As used herein, "dementia" refers to a neurodegenerative disease characterized by chronic loss of mental capacity, particularly progressive deterioration of thinking and/or memory and/or behavior and/or personality. Examples of neurodegenerative diseases causing dementia include, but are not limited to, frontotemporal dementia (FTD), behavioral variant FTD, progressive non-fluent aphasia, semantic dementia, frontotemporal dementia associated with amyotrophic lateral sclerosis (FTD-ALS), frontotemporal lobar degeneration (FTLD), frontotemporal lobar degeneration associated with amyotrophic lateral sclerosis (FTLD-ALS), Parkinson-dementia, Lewy bodies dementia, progressive supranuclear palsy, corticobasal degeneration syndrome and Alzheimer's disease.

In one embodiment, said neurodegenerative disease is a motor neuron disease. As used herein, a "motor neuron disease" refers to a neurodegenerative disease characterized by the dysfunction and/or death of motor neurons, such as, for example, upper motor neurons and lower motor neurons. Example of motor neuron diseases include, but are not limited to, ALS, FTD-ALS, FTLD-ALS, and primary lateral sclerosis.

In one embodiment, said neurodegenerative disease is a parkinsonian syndrome. Examples of parkinsonian syndromes include, but are not limited to, typical parkinsonian syndromes, such as, for example, Parkinson's disease and Parkinson's dementia; and atypical parkinsonian syndromes, such as, for example, progressive supranuclear palsy, corticobasal degeneration syndrome, Lewy bodies dementia and multiple system atrophy.

In one embodiment said neurodegenerative disease is frontotemporal dementia (FTD), frontotemporal lobar degeneration (FTLD), amyotrophic lateral sclerosis (ALS), FTD-ALS or FTLD-ALS.

Examples of body fluids include, but are not limited to, whole blood, plasma, serum, bile, lymph, pleural fluid, semen, saliva, sweat, urine and cerebrospinal fluid.

Preferably, said body fluid is plasma or cerebrospinal fluid.

Methods for determining a protein level in a body fluid are well-known in the art. Examples of such methods include, but are not limited to, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA) and the like.

Antibodies directed to the C9ORF72 protein are commercially available, such as, for example, S-14 (Santa-Cruz Biotechnology) or Q96LT7 (Gene Tex).

A reference value can be relative to a number or value derived from population studies, including without limitation, such subjects having similar age range, subjects in the same or similar ethnic group, and the like.

In one embodiment, the reference value is constructed using algorithms and other methods of statistical and structural classification.

In one embodiment of the invention, the reference value is derived from the measurement of the protein level of C9ORF72 in a control sample derived from one or more substantially healthy subjects. As used herein, a "substantially healthy subject" has not been previously diagnosed or identified as having or suffering from a neurodegenerative disease.

In another embodiment of the invention, the reference value is derived from the measurement of the protein level of C9ORF72 in a control sample derived from one or more subjects having no hexanucleotide repeat expansion in the C9ORF72 gene.

In one embodiment of the invention, the reference value is derived from the measurement of the protein level of C9ORF72 in a reference population.

In one embodiment, the reference population comprises substantially healthy subjects, preferably at least 100, more preferably at least 250, more preferably at least 500 substantially healthy subjects.

In another embodiment, the reference population comprises subjects having no hexanucleotide repeat expansion in the C9ORF72 gene, preferably at least 100, more preferably at least 250, more preferably at least 500 subjects having no hexanucleotide repeat expansion in the C9ORF72 gene.

In one embodiment, the reference value corresponds to the mean value of the protein level of C9ORF72 measured in the reference population.

In one embodiment of the invention, the reference value corresponds to the median value of the protein level of C9ORF72 in the reference population.

In one embodiment of the invention where the method of the invention is for monitoring the progression of a neurodegenerative disease in a patient, the reference value is the level of C9ORF72 protein previously measured in a body fluid of the same subject, such as, for example, measured one month before, preferably six months before, more preferably one year before or more.

In one embodiment, a reduced level of C9ORF72 protein in a body fluid of a subject as compared to the reference value is indicative of the presence of a neurodegenerative disease in said subject. In one embodiment, a reduced level of C9ORF72 protein in a body fluid of a subject as compared to the reference value is indicative of an increased risk of said subject to develop a neurodegenerative disease. In one embodiment, a reduced level of C9ORF72 protein in a body fluid of a subject as compared to the reference value is indicative of a predisposition of said subject to a neurodegenerative disease.

In one embodiment, a reduced level corresponds to a percent reduction of 5% compared to the reference value, preferably a percent reduction of 10%, 15%, 20%, 25%, 30%, 40%, 50% or more.

In one embodiment, the subject is an animal, preferably a mammal, more preferably a human.

In one embodiment, the subject presents a familial history of neurodegenerative disease.

In another embodiment, the subject does not present any familial history of neurodegenerative disease.

In one embodiment, the subject is substantially healthy.

In one embodiment of the invention, the subject has been diagnosed with dementia, such as, for example, with FTD or FTLD. According to this embodiment, the method of the invention is a method for diagnosing a motor neuron disease in said subject, or determining a predisposition of said subject to a motor neuron disease or determining an increased risk of said subject of developing a motor neuron disease. Preferably, said motor neuron disease is ALS. Accordingly, the method of the invention may be a method for diagnosing ALS or for determining a predisposition to ALS or determining an increased risk of developing ALS in a FTD or FTLD patient.

In one embodiment of the invention, the subject has been diagnosed with a motor neuron disease, such as, for example, with ALS. According to this embodiment, the method of the invention is a method for diagnosing dementia in said subject, or determining a predisposition of said subject to dementia or determining an increased risk of said subject of developing dementia. Preferably, said dementia is FTD or FTLD. Accordingly, the method of the invention may be a method for diagnosing FTD or FTLD or for determining a predisposition to FTD or FTLD or determining an increased risk of developing FTD or FTLD in an ALS patient.

In one embodiment of the invention, the method is for monitoring the response to a treatment in a subject having or being at risk of having a neurodegenerative disease, wherein said method comprises determining the protein level of C9ORF72 in a body fluid of the subject, and comparing said protein level to a reference value.

In said embodiment, the reference value is the level of C9ORF72 protein previously measured in a body fluid of the same subject, such as, for example, measured before treatment and at different time points after or during the treatment, preferably 1 month after beginning of the treatment and/or 2, 3, 4, 5, 6, 8, 10, and/or 12 months after beginning of the treatment.

In one embodiment, the method of the invention is for diagnosing presenile dementia, or for determining a predisposition to presenile dementia or determining an increased risk of developing presenile dementia. As used herein, "presenile dementia" refers to dementia characterized by the onset of clinically detectable symptoms of dementia before a subject is 65 years of age.

According to this embodiment, the subject is under 65 years old, preferably under 60, 50, 40 years old.

The present invention also relates to a method for classifying a subject as having a neurodegenerative disease or a predisposition of developing a neurodegenerative disease or as being at risk of developing a neurodegenerative disease, wherein said method comprises determining the protein level of C9ORF72 in a body fluid of the subject, and comparing said protein level to a reference value.

The present invention also relates to a method for determining the risk of a subject of developing a neurodegenerative disease, or for determining the risk of disease onset, or for monitoring the progression of a neurodegenerative disease, wherein said method comprises determining the protein level of C9ORF72 in a body fluid of the subject, and comparing said protein level to a reference value, wherein said subject carries a C9ORF72 hexanucleotide repeat expansion or presents a familial history of neurodegenerative disease or belongs to a kindred wherein at least one member carries a C9ORF72 hexanucleotide repeat expansion.

The inventors herein show (see Examples), that the decrease of the protein level of C9ORF72 in a body fluid of a subject carrying a C9ORF72 hexanucleotide repeat expansion is associated with the presence of disease symptoms. Indeed, said protein level is not decreased in asymptomatic subjects carrying a C9ORF72 hexanucleotide repeat expansion. The inventors therefore suggest that determining the protein level of C9ORF72 in a body fluid of the subject may allow predicting the date of clinical onset. Therefore, it may also allow scheduling the treatment of the neurodegenerative disease. Indeed, in order to be the most efficient, said treatment should be administered shortly before symptoms onset.

The present invention also relates to a method for diagnosing a neurodegenerative disease in a subject, or determining a predisposition of a subject to a neurodegenerative disease or determining an increased risk of a subject of developing a neurodegenerative disease, or predicting the time of development of clinical symptoms, wherein said method comprises:
(a) determining the protein level of C9ORF72 in a body fluid of the subject, and comparing said protein level to a reference value; and
(b) optionally assessing the presence of a hexanucleotide repeat expansion in the C9ORF72 gene of said subject.

In one embodiment of the invention, step (b) of the method is carried out if a reduced level of C9ORF72 protein in a body fluid as compared to a reference value was determined at step (a).

In one embodiment of the invention, the presence of a hexanucleotide repeat expansion in the C9ORF72 gene of said subject is assessed by repeat-primed PCR or by Southern Blot. Preferably, said assessment is carried out on a genomic DNA sample of the subject.

The present invention thus also relates to a prescreening method for identifying subjects for whom the assessment of the presence of a hexanucleotide repeat expansion in the C9ORF72 gene may be beneficial, said method comprising determining the protein level of C9ORF72 in a body fluid of the subject, and comparing said protein level to a reference value.

The method of the present invention thus presents the advantages of being rapid to implement, to be inexpensive and to present sufficient sensitivity to detect subjects for who the assessment of a hexanucleotide repeat expansion may be beneficial.

### BRIEF DESCRIPTION OF THE DRAWINGS

**Figure 1** is a picture of a western blot membrane, showing the detection of C9ORF72 protein in the cerebrospinal fluid (wells 1 to 3) and in the plasma (wells 5 to 10) of FTD patients. 4: molecular weight (MW) marker.
**Figure 2** is a graph showing the detection of C9ORF72 by Sandwich Elisa (detection at 450 nm) in a cerebrospinal fluid (CSF) sample of a control subject (Control) and of a FTD patient carrying an hexanucleotide repeat expansion in the C9ORF72 gene (Hex Rep), in function of the dilution factor of the CSF sample.
**Figure 3** is a graph showing the detection of C9ORF72 by Sandwich Elisa (detection at 450 nm) in a CSF sample of a FTD patient having no hexanucleotide repeat expansion in the C9ORF72 gene (Without hexanucleotide repeat) and in a FTD patient carrying an hexanucleotide repeat expansion in the C9ORF72 gene (With hexanucleotide repeat), in function of the dilution factor of the CSF sample.
**Figure 4** is a graph showing the protein level of C9ORF72 in plasma from a control subject (Control), or from symptomatic (MUT symptom) or asymptomatic (MUT asympto) patient carrying a C9ORF72 hexanucleotide repeat expansion.

### EXAMPLES

The present invention is further illustrated by the following examples.

### Material and methods

### Western blot analysis of C9orf72 protein:

In order to check the presence of C9orf72 protein in human body fluids, a western blot analysis on 12.5% PAGE was performed using plasma and cerebrospinal fluid (CSF) from patients with FTD carrying or not C9orf72 hexanucleotide expansion.

Proteins from plasma or CSF were separated on 12% Tris-glycine polyacrylamide gels. For immunoblotting, proteins separated by SDS-PAGE were transferred to transfer PVDF membrane (0,45 µM, Millipore). The membrane was blocked with 5% dried skimmed milk in 10 mM Tris-HCl, 150 mM NaCl, 0.05% Tween 20 pH 7.5 (TBS-T) overnight at +4°C. After blocking, the membrane was incubated for 2 h with antiC9orf72 rabbit polyclonal antibodies S-14 from Santa-Cruz Biotechnology or Q96LT7 from Gene Tex diluted to 1:1000 in TBS-T. The membranes were washed three times with TBS-T, followed by incubation with a secondary antibody, HRP-conjugated goat anti-rabbit antibody (Sigma) diluted to 1:5000. The protein bands were visualized by using enhanced chemiluminescence reagent (ECL).

### Immunoassay protocol

In order to quantify the detected C9orf72 protein in body fluids a sandwich ELISA system was performed. Because all the available commercial antibodies directed against the target protein were obtained from the same species (rabbit), the S-14 antibody was conjugated to Horse Radish Peroxidase (HRP), and used as the detection antibody in a sandwich ELISA system.

The Elisa plates (Nunc Maxisorp 96-well) were coated by overnight incubation at 4°C with 2 µg/ml anti-C9orf72 polyclonal antibody raised against recombinant protein fragment corresponding to a region within amino acids 1 and 58 (Q96LT7 - Gene Tex), 100 µl/well diluted in 200 mM NaHCO₃ buffer pH 9,6. The plates were washed three times with PBST (0.01 M phosphate buffer, 0.0027 M KCl and 0.137 M NaCl, pH 7.4 containing 0.05% Tween 20) and incubated with 300 µl blocking buffer (PBST containing 5% dried skimmed milk) for 2 h at 37°C. The plates were washed again 3 times with PBST, and 100 µl of a serial dilution of a plasma samples were added to each well, in order to check the signal response of the method. To eliminate inter-assay variability as a confounding factor, all CSF samples were run in triplicate on the same day. After washing three times with PBST, the detection HRP-conjugated S-14 antibody (Santa Cruz), 100 µl/well, diluted to 0.2 µg/ml in blocking buffer, was added and the plate was incubated at 37°C for 2h. After washing 5 times with PBST, 100 µl/well of trimethyl -benzidine (Sigma) was added to each well and incubated for 30 minutes. The peroxidase reaction was stopped by adding 50 µl/well of 0.6 M H₂SO₄ solution. The absorbance of developed coloration was measured by a plate reader at 450 nm.

### Results

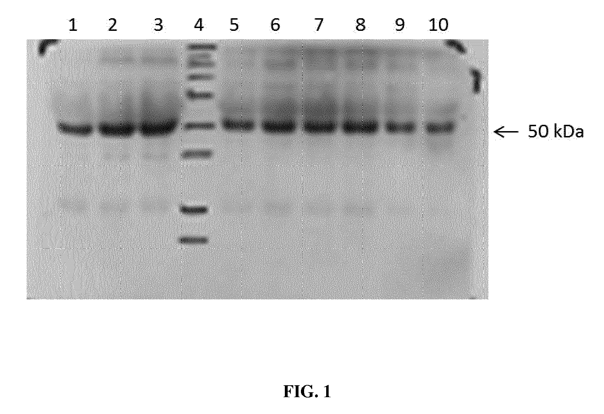

As shown in **Figure 1**, immunoblotting of proteins from undiluted CSF or 1:10 diluted plasma showed a specific band at 50 kDa visualized by using both commercial S-14 (Santa-Cruz) and Q96LT7 (Gene Tex) antibodies. The bands were more intense in plasmas than in CSF.

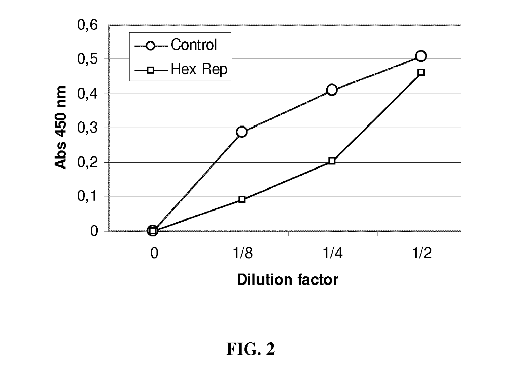

Plasmas from a control subject and from a FTD patient with hexanucleotide repeat expansion in the C9orf72 gene have been diluted and analyzed by sandwich ELISA. Results are shown in **Figure 2**. Results indicate a proportional response according to the sample dilution. The absorbance of plasma from the patient (Hex Rep) is lower than that observed with plasma from control subject (Control).

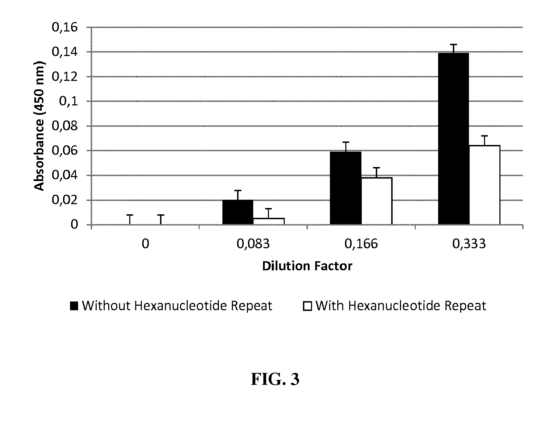

We then compared the C9ORF72 level in two FTD patients having a familial form of ALS-FTD, wherein the first one carries the hexanucleotide repeat expansion in the C9orf72 gene whereas the second one does not (**Figure 3**). As shown in **Figure 3**, a significant decrease in absorbance in a sample obtained from the patient with hexanucleotide repeat expansion is observed as compared to the patient without the expansion.

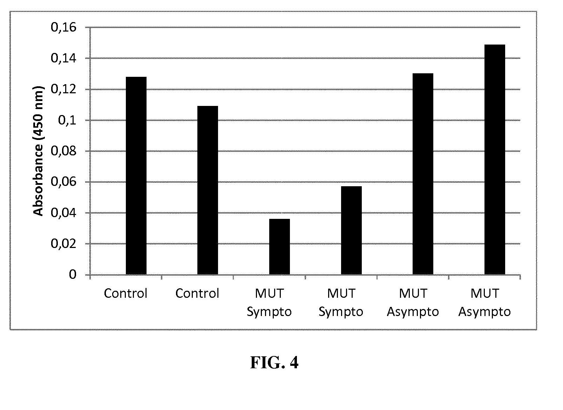

Using the developed ELISA method, the protein level of C9ORF72 in the plasma was compared between subjects carrying C9ORF72 hexanuleotide repeat expansion with or without ALS-FTD symptoms (**Figure 4**). As shown in **Figure 4**, the protein level of C9ORF72 in the plasma was decreased in symptomatic patients, but not in asymptomatic patients, as compared to controls (healthy).

This result suggests that the protein level of C9ORF72 in the plasma may be a predictive marker in the development of ALS-FTD symptoms in subjects with C9ORF72 hexanucleotide repeat expansion.

## Claims

1. A method for diagnosing a neurodegenerative disease in a subject, or determining a predisposition of a subject to a neurodegenerative disease or determining an increased risk of a subject of developing a neurodegenerative disease, wherein said method comprises determining the protein level of C9ORF72 in a body fluid of the subject, and comparing said protein level to a reference value.

2. The method according to claim **1,** wherein said body fluid is selected from plasma or cerebrospinal fluid.

3. The method according to claim **1** or claim **2,** wherein the protein level is measured by immunoassay, preferably by ELISA.

4. The method according to anyone of claims **1** to **3,** wherein a reduced protein level as compared to the reference value is indicative of the presence of, of a predisposition to or of an increased risk of the subject developing a neurodegenerative disease.

5. The method according to anyone of claims **1** to **4,** wherein the neurodegenerative disease is frontotemporal dementia, progressive non-fluent aphasia, semantic dementia, frontotemporal dementia associated with amyotrophic lateral sclerosis, frontotemporal lobar degeneration associated with amyotrophic lateral sclerosis, amyotrophic lateral sclerosis without dementia, spastic paraplegia, primary lateral sclerosis, schizophrenia, psychotic disorders, Alzheimer's disease, idiopathic Parkinson, Parkinson-dementia, preogressive supranuclear palsy, corticobasal degeneration syndromes, Lewy bodies dementia and multiple system atrophy.

6. The method according to anyone of claims **1** to **5,** wherein the degenerative disease is frontotemporal dementia (FTD), frontotemporal lobar degeneration (FTLD), amyotrophic lateral sclerosis (ALS), FTD-ALS or FTLD-ALS.

7. The method according to anyone of claims **1** to **6,** wherein the subject presents a familial history of neurodegenerative disease.

8. The method according to anyone of claims **1** to **6,** wherein the subject does not present any familial history of neurodegenerative disease.

9. A method for classifying a subject as having a neurodegenerative disease or a predisposition of developing a neurodegenerative disease or as being at risk of developing a neurodegenerative disease, wherein said method comprises determining the protein level of C9ORF72 in a body fluid of the subject, and comparing said protein level to a reference value.

10. A prescreening method for identifying subjects for whom the assessment of the presence of a hexanucleotide repeat expansion in the C9ORF72 gene may be beneficial, said method comprising determining the protein level of C9ORF72 in a body fluid of the subject, and comparing said protein level to a reference value.

11. A method for determining the risk of neurodegenerative disease onset in a subject, wherein said method comprises determining the protein level of C9ORF72 in a body fluid of the subject, and comparing said protein level to a reference value.

12. A method for monitoring the response to a treatment in a subject having or being at risk of having a neurodegenerative disease, wherein said method comprises determining the protein level of C9ORF72 in a body fluid of the subject, and comparing said protein level to a reference value.

13. A method for monitoring the progression of a neurodegenerative disease in a subject carrying a C9ORF72 hexanucleotide repeat expansion, wherein said method comprises determining the protein level of C9ORF72 in a body fluid of the subject, and comparing said protein level to a reference value.

14. The method according to anyone of claims **1** to **13,** wherein the reference value is derived from the measurement of the protein level of C9ORF72 in a control sample derived from one or more substantially healthy subjects.

15. The method according to anyone of claims **1** to **13,** wherein the reference value corresponds to the level of C9ORF72 protein previously measured in a body fluid of said subject.


SEQUENCE LISTING

<110> ICM (INSTITUT DU CERVEAU ET DE LA MOÃ\213LLE Ã\211PINIÃ\210RE)
      LE BER, Isabelle

<120> PROTEIN LEVEL OF C9ORF72 FOR DIAGNOSING A NEURODEGENERATIVE DISEASE

<130> CV - 210/EP

<160> 2

<170> BiSSAP 1.0

<210> 1
<211> 1446
<212> DNA
<213> Homo sapiens

<220>
<221> source
<222> 1..1446
<223> /mol_type="DNA"
      /organism="Homo sapiens"

<400> 1
atgtcgactc tttgcccacc gccatctcca gctgttgcca agacagagat tgctttaagt      60

ggcaaatcac ctttattagc agctactttt gcttactggg acaatattct tggtcctaga     120

gtaaggcaca tttgggctcc aaagacagaa caggtacttc tcagtgatgg agaaataact     180

tttcttgcca accacactct aaatggagaa atccttcgaa atgcagagag tggtgctata     240

gatgtaaagt tttttgtctt gtctgaaaag ggagtgatta ttgtttcatt aatctttgat     300

ggaaactgga atggggatcg cagcacatat ggactatcaa ttatacttcc acagacagaa     360

cttagtttct acctcccact tcatagagtg tgtgttgata gattaacaca tataatccgg     420

aaaggaagaa tatggatgca taaggaaaga caagaaaatg tccagaagat tatcttagaa     480

ggcacagaga gaatggaaga tcagggtcag agtattattc caatgcttac tggagaagtg     540

attcctgtaa tggaactgct ttcatctatg aaatcacaca gtgttcctga agaaatagat     600

atagctgata cagtactcaa tgatgatgat attggtgaca gctgtcatga aggctttctt     660

ctcaatgcca tcagctcaca cttgcaaacc tgtggctgtt ccgttgtagt aggtagcagt     720

gcagagaaag taaataagat agtcagaaca ttatgccttt ttctgactcc agcagagaga     780

aaatgctcca ggttatgtga agcagaatca tcatttaaat atgagtcagg gctctttgta     840

caaggcctgc taaaggattc aactggaagc tttgtgctgc ctttccggca agtcatgtat     900

gctccatatc ccaccacaca catagatgtg gatgtcaata ctgtgaagca gatgccaccc     960

tgtcatgaac atatttataa tcagcgtaga tacatgagat ccgagctgac agccttctgg    1020

agagccactt cagaagaaga catggctcag gatacgatca tctacactga cgaaagcttt    1080

actcctgatt tgaatatttt tcaagatgtc ttacacagag acactctagt gaaagccttc    1140

ctggatcagg tctttcagct gaaacctggc ttatctctca gaagtacttt ccttgcacag    1200

tttctacttg tccttcacag aaaagccttg acactaataa aatatataga agacgatacg 1260

cagaagggaa aaaagccctt taaatctctt cggaacctga agatagacct tgatttaaca 1320

gcagagggcg atcttaacat aataatggct ctggctgaga aaattaaacc aggcctacac 1380

tcttttatct ttggaagacc tttctacact agtgtgcaag aacgagatgt tctaatgact 1440

ttttaa 1446

<210> 2
<211> 481
<212> PRT
<213> Homo sapiens

<220>
<221> SOURCE
<222> 1..481
<223> /mol_type="protein"
/organism="Homo sapiens"

<400> 2
Met Ser Thr Leu Cys Pro Pro Pro Ser Pro Ala Val Ala Lys Thr Glu
1               5                   10                  15
Ile Ala Leu Ser Gly Lys Ser Pro Leu Leu Ala Ala Thr Phe Ala Tyr
            20                  25                  30
Trp Asp Asn Ile Leu Gly Pro Arg Val Arg His Ile Trp Ala Pro Lys
        35                  40                  45
Thr Glu Gln Val Leu Leu Ser Asp Gly Glu Ile Thr Phe Leu Ala Asn
    50                  55                  60
His Thr Leu Asn Gly Glu Ile Leu Arg Asn Ala Glu Ser Gly Ala Ile
65                  70                  75                  80
Asp Val Lys Phe Phe Val Leu Ser Glu Lys Gly Val Ile Ile Val Ser
                85                  90                  95
Leu Ile Phe Asp Gly Asn Trp Asn Gly Asp Arg Ser Thr Tyr Gly Leu
            100                 105                 110
Ser Ile Ile Leu Pro Gln Thr Glu Leu Ser Phe Tyr Leu Pro Leu His
        115                 120                 125
Arg Val Cys Val Asp Arg Leu Thr His Ile Ile Arg Lys Gly Arg Ile
    130                 135                 140
Trp Met His Lys Glu Arg Gln Glu Asn Val Gln Lys Ile Ile Leu Glu
145                 150                 155                 160
Gly Thr Glu Arg Met Glu Asp Gln Gly Gln Ser Ile Ile Pro Met Leu
                165                 170                 175
Thr Gly Glu Val Ile Pro Val Met Glu Leu Leu Ser Ser Met Lys Ser
            180                 185                 190
His Ser Val Pro Glu Glu Ile Asp Ile Ala Asp Thr Val Leu Asn Asp
        195                 200                 205
Asp Asp Ile Gly Asp Ser Cys His Glu Gly Phe Leu Leu Asn Ala Ile
    210                 215                 220
Ser Ser His Leu Gln Thr Cys Gly Cys Ser Val Val Val Gly Ser Ser
225                 230                 235                 240
Ala Glu Lys Val Asn Lys Ile Val Arg Thr Leu Cys Leu Phe Leu Thr
                245                 250                 255
Pro Ala Glu Arg Lys Cys Ser Arg Leu Cys Glu Ala Glu Ser Ser Phe
            260                 265                 270
Lys Tyr Glu Ser Gly Leu Phe Val Gln Gly Leu Leu Lys Asp Ser Thr
        275                 280                 285
Gly Ser Phe Val Leu Pro Phe Arg Gln Val Met Tyr Ala Pro Tyr Pro
    290                 295                 300
Thr Thr His Ile Asp Val Asp Val Asn Thr Val Lys Gln Met Pro Pro
305                 310                 315                 320
Cys His Glu His Ile Tyr Asn Gln Arg Arg Tyr Met Arg Ser Glu Leu
                325                 330                 335

Thr Ala Phe Trp Arg Ala Thr Ser Glu Glu Asp Met Ala Gln Asp Thr
            340                 345                 350
Ile Ile Tyr Thr Asp Glu Ser Phe Thr Pro Asp Leu Asn Ile Phe Gln
        355                 360                 365
Asp Val Leu His Arg Asp Thr Leu Val Lys Ala Phe Leu Asp Gln Val
    370                 375                 380
Phe Gln Leu Lys Pro Gly Leu Ser Leu Arg Ser Thr Phe Leu Ala Gln
385                 390                 395                 400
Phe Leu Leu Val Leu His Arg Lys Ala Leu Thr Leu Ile Lys Tyr Ile
                405                 410                 415
Glu Asp Asp Thr Gln Lys Gly Lys Lys Pro Phe Lys Ser Leu Arg Asn
            420                 425                 430
Leu Lys Ile Asp Leu Asp Leu Thr Ala Glu Gly Asp Leu Asn Ile Ile
        435                 440                 445
Met Ala Leu Ala Glu Lys Ile Lys Pro Gly Leu His Ser Phe Ile Phe
    450                 455                 460
Gly Arg Pro Phe Tyr Thr Ser Val Gln Glu Arg Asp Val Leu Met Thr
465                 470                 475                 480
Phe